(19) Europäisches Patentamt
European Patent Office
Office européen des brevets

(11) Publication number: 0 424 179 A2

# (12) EUROPEAN PATENT APPLICATION

(21) Application number: 90311526.9

(22) Date of filing: 19.10.90

(51) Int. Cl.[5]: A61K 31/445, A61K 31/215, A61K 31/40, A61K 31/135

(30) Priority: 20.10.89 US 424548
18.01.90 US 467139

(43) Date of publication of application:
24.04.91 Bulletin 91/17

(84) Designated Contracting States:
AT BE CH DE DK FR GB IT LI LU NL SE

(71) Applicant: Olney, John William
1 Lorenzo Lane
St. Louis, Missouri 63124(US)

(72) Inventor: Olney, John William
1 Lorenzo Lane
St. Louis, Missouri 63124(US)

(74) Representative: Woods, Geoffrey Corlett et al
J.A. KEMP & CO. 14 South Square Gray's Inn
London WC1R 5EU(GB)

## (54) Use of combined excitatory amino acid and cholinergic antagonists to prevent neurological deterioration.

(57) The neurotoxic effects of NMDA antagonists are reduced by administering, in conjunction with an NMDA antagonist, an anti-cholinergic agent which is capable of penetrating the blood-brain barrier and which exerts a pharmaceutically antagonistic effect on cholinergic receptors of the muscarinic type on the surfaces of neurons in the central nervous system. Also, excitotoxic damage to neurons in a mammalian central nervous system is reduced by the administration of:

(a) an NMDA antagonist which penetrates blood-brain barriers in quantities sufficient to inhibit the functioning of NMDA receptors; and

(b) a non-NMDA antagonist which penetrates blood-brain barriers in quantities sufficient to inhibit the functioning of at least one type of non-NMDA receptor.

EP 0 424 179 A2

# USE OF COMBINED EAA AND CHOLINERGIC ANTAGONISTS TO PREVENT NEUROLOGICAL DETERIORATION

This invention is in the fields of pharmacology and neurology. It relates to compounds and methods for protecting the central nervous system against neurotoxic side effects of certain therapeutic drugs and against neurodegenerative disease processes.

## Receptors, messenger molecules, agonists, and antagonists

The surfaces of nerve cells in the central nervous system (the CNS, which includes the brain, spinal cord, and retina) contain various types of receptor molecules. In general, a receptor molecule is a polypeptide which straddles a cell membrane. When a messenger molecule interacts with the exposed extracellular portion of the membrane receptor molecule, it triggers a difference in the electrochemical status of the intracellular portion of the receptor, which in turn provokes some response by the cell. The messenger molecule does not bond to the receptor; instead, it usually disengages from the receptor after a brief period and returns to the extracellular fluid. Most receptor molecules are named according to the messenger molecules which bind to them.

An "agonist" is any molecule, including the naturally occurring messenger molecule, which can temporarily bind to and activate a certain type of receptor. An agonist can cause the same effect as the natural messenger molecule, or in some cases it can cause a more intense effect (for example, if it has a tighter affinity for the receptor molecule and remains bound to the receptor for a prolonged period).

By contrast, an "antagonist" is a molecule which can block or reduce the effects exerted by the natural messenger molecule. This can happen in several different ways. A "competitive antagonist" binds to a certain type of receptor without triggering it, thereby preventing the natural messenger molecule from reaching and activating the receptor. A "non-competitive antagonist" functions in other ways. For example, a receptor referred to as the PCP receptor, which is triggered by molecules such as PCP or MK-801, apparently can override the effects of a different type of receptor, the NMDA receptor (both receptors are discussed below). Therefore, PCP and MK-801 are regarded as non-competitive antagonists for the NMDA receptor.

The role a certain molecule plays as an agonist or antagonist must be viewed with regard to a certain type of receptor. For example, while MK-801 is an antagonist for the NMDA receptor, it is an agonist for the PCP receptor. Most agonists and antagonists are xenobiotic drugs, i.e, they do not exist naturally in the body.

For more information on neuroanatomy, neurotransmitters, receptor molecules, and agonists and antagonists which interact with CNS receptors, see Adelman 1987 (complete cita tions are provided below).

The two main classes of excitatory receptor molecules are referred to as cholinergic receptors and glutamate receptors. Glutamate (Glu) receptors are also referred to as "excitatory amino acid" or "EAA" receptors. Both types of receptors are present in the synaptic junctions that serve as pathways for impulses between CNS nerve cells. Most other types of receptors in the CNS involve inhibitory neurotransmitters.

## Excitatory amino acids and neurotoxicity

Accumulating evidence implicates excitatory amino acids (EAA's) as causative agents in certain types of CNS damage associated with hypoxia and ischemia. Hypoxia refers to detrimentally low levels of oxygen, while ischemia refers to detrimentally low blood flow. Those conditions are obviously related; any factor that causes ischemia (such as stroke or cardiac arrest) will also cause hypoxia as a direct result. They are referred to herein as hypoxia/ischemia, which refers to hypoxia and/or ischemia. Hypoxia can also be caused by various factors not involving ischemia, such as asphyxia, anemia, or carbon monoxide poisoning.

EAA's are also implicated in CNS damage relating to various other problems or conditions, including epsilepsy, hypoglycemia, alcoholism, and trauma of the brain or spinal cord. It is also believed that EAA's may be involved in slowly developing neurodegenerative disorders such as Huntington's, Parkinson's and Alzheimer's diseases.

The two primary EAA's, glutamate and aspartate (the ions or salts of glutamic acid and aspartic acid), are found naturally in high concentrations in the CNS, where they function as excitatory neurotransmitters. Although these substances are beneficial and of critical importance for the normal functioning of the CNS,

under abnormal conditions they can damage or destroy CNS neurons by an "excitotoxic" process. Excitotoxicity refers to the process whereby EAA's that are released by one neuron excessively stimulate (excite) receptor molecules located on the external surface of another neuron. Excitotoxicity also refers to the same excitatory neurotoxic process when the EAA receptors are triggered by glutamate (or analogs of glutamate) ingested in foods or administered systemically to various mammalian species. Glutamate and asparate are sometimes called "endogenous" excitotoxins, meaning that they are excitatory neurotoxins contained naturally within the CNS, whereas EAA ingested in foods or administered systemically are referred to as "exogenous" excitotoxins. For an extensive review, see Olney 1989a.

EAA receptors, also known as glutamate receptors, are categorized into three subtypes, each named after a glutamate analog which selectively excites them: N-methyl-D-aspartate (NMDA), kainic acid (KA), and quisqualate (QUIS). Glutamate is capable of activating all three receptor subtypes.

Normally, relatively high glutamate concentrations (in the general range of about 10 mM) are maintained inside cells of the CNS, but high concentrations are not allowed in the extracellular fluid where glutamate can exert excitotoxic action at EAA receptors. After glutamate is released in small amounts by a neuron for neurotransmitter purposes, it normally is transported back inside a cell very efficiently by means of a transport mechanism that requires energy.

Under severe low energy conditions such as hypoglycemia, hypoxia, or ischemia, the transport systems may lack sufficient energy to transfer glutamate back into the cell, so the glutamate accumulates outside the cell at abnormal levels and excessively stimulates the EAA receptors. This can lead to continuous neuronal discharge, which in turn causes additional glutamate release and extracellular accumulation of excess glutamate, leading to a cascade of increasing neurotoxic injury, which can result in permanent brain damage or death.

This type of glutamate cascade can induce one form of "excitotoxicity," which refers to any stimulation of CNS neurons, via EAA receptors, which damages the neurons. Other mechanisms by which EAA's can cause neuronal injury include abnormal sensitivity of EAA receptors to the excitatory action of EAA's, and the presence of abnormal molecules (such as glutamate analogs, certain types of food poisons, etc.) with excitotoxic properties. Such receptor-triggering molecules can accumulate at EAA receptors because they are not recognized by the cellular transport systems as molecules which should be removed from the extracellular fluid.

In these neurotoxic situations, one method of preventing or minimizing excitotoxic injury to the neurons involves administering drugs that selectively block or "antagonize" the action of the excitotoxic molecules at the EAA receptors.

## Non-competitive NMDA antagonists

The EAA receptor subtype that has been implicated most frequently in neurodegenerative diseases and neurotoxicity is the NMDA receptor. An entire issue of *Trends in Neurosciences* (Vol. 10, Issue 7, July 1987) was devoted to review articles pertaining to the NMDA receptor, and to NMDA "antagonists" (i.e., molecules which can block or reduce the effects of NMDA at NMDA receptors).

The most powerful and effective NMDA antagonists known at the present time act at another receptor, the phencyclidine (PCP) receptor, which is considered a component of an ion channel complex that involves the NMDA receptor (Kemp et al 1987). Compounds that activate the PCP receptor are called non-competitive NMDA antagonists, because they do not compete for binding sites at NMDA receptors. When phencyclidine or its analogs activate a PCP receptor, the flow of ions through the NMDA ion channel is blocked or substantially reduced, so that when the NMDA receptor is activated by an EAA, the NMDA receptor response does not result in the flow of ion currents. This blocks the excitation of the neuron.

Four compounds which can activate the PCP receptor, and which therefore serve as non-competitive NMDA antagonists, are phencyclidine, MK-801, ketamine, and tiletamine. All four can penetrate the blood-brain barrier.

Phencyclidine (PCP) was originally introduced into clinical medicine some 30 years ago as an anesthetic (Goodman and Gilman 1975). Shortly thereafter, it was withdrawn from the market because it was found to have hallucinogenic properties which invited illicit use by drug abusers. Since then, PCP (also known as angel dust) has become popular as a "recreational" drug and is a major cause of drug-induced psychotic reactions (which occasionally lead to extremely violent crimes) among drug abusers. It was recently shown (Olney et al 1989c) that PCP can cause morphological damage in the brain (vacuole formation and mitochondrial dissolution in cingulate and retrosplenial cerebrocortical neurons). This neurotoxic effect, which is discussed in greater detail below, may be related to the mechanism by which PCP

causes toxic psychoses.

Ketamine, a drug manufactured by Parke Davis and marketed under the trade name Ketalar, is currently used both in human and in veterinary medicine as an anesthetic. Ketamine is known to activate PCP receptors and, like PCP and MK-801, is recognized as a non-competitive antagonist of the NMDA receptor-ion channel complex (Kemp et al 1987). Ketamine was among the drugs recently shown to produce pathomorphological effects on cingulate/retrosplenial neurons following administration to rats (Olney et al 1989c). Ketamine is also known to induce an acute transient psychosis (called an "emergence" reaction) in a substantial portion of human patients anesthetized with this agent (Physicians Desk Reference, 1986). It has been proposed that the psychotic effects of ketamine, like those of PCP, may be psychological manifestations of the same toxic process that causes pathomorphological changes in cingulate/retrosplenial neurons, in which case a drug that could prevent the pathomorphological changes might also prevent or reduce the psychotic manifestations.

Tiletamine is a drug manufactured by A.H. Robins. It is currently used in veterinary medicine, and is widely used for anesthesia on house pets. Tiletamine, like PCP, MK-801 and ketamine, is known to activate PCP receptors and is recognized as a non-competitive antagonist of the NMDA receptor-ion channel complex. Tiletamine was among the drugs recently shown to produce pathomorphological effects on cingulate/retrosplenial oortical neurons following ip administration to rats (Olney et al 1989c).

MK-801, a phencyclidine analog manufactured by Merck, Sharp and Dohme (Rahway, NJ) and referred to as dizocilpine, was initially proposed as an anticonvulsant, but after brief human clinical trials, it was withdrawn from further testing several years ago with no published explanation. At about the same time, it was discovered that PCP receptors are an integral component of the NMDA receptor ion channel complex (Kemp et al 1987), and that MK-80 1 is a potent activator of PCP receptors, with higher affinity for the PCP binding site than PCP itself. MK-801 is more specific for PCP receptors than any other known compound; in *in vitro* studies using chick embryo retina tissue, it was shown that MK-801 is approximately 5-10 times more powerful than PCP in preventing the neurotoxic effects of NMDA on retinal neurons (Olney et al 1987). Previously, PCP had been recognized as the most powerful known antagonist of NMDA neurotoxicity (Olney et al 1986). Unfortunately, MK-801 also mimics PCP in causing pathomorphological effects on cingulate/retrosplenial cortical neurons (Olney et al 1989c).

Because of its great potency and the ease with which it penetrates blood brain barriers, MK-801 has become the drug used most widely in animal experiments aimed at testing the neuroprotective properties of NMDA antagonists. Since it has now been shown to protect CNS neurons against various degenerative processes that are thought to involve excessive activation of NMDA receptors (e.g., hypoxia/ischemia, prolonged seizures, hypoglycemia, thiamine deficiency, head or spinal cord trauma), there is considerable interest in using MK-801 for neuroprotective purposes in clinical neurology. Clearly, it would be desirable to have a means of preventing the toxic action of MK-801 on cingulate/retrosplenial cortical neurons, thereby making this drug available for human therapy with reduced risk of neurotoxic side effects.

Other non-competitive NMDA antagonists which apparently do not function at the PCP receptor have recently been described, including glycine antagonists and polyamine antagonists, which exert blocking action at glycine and polyamine receptors, respectively, both of which are coupled to the NMDA receptor ion-channel complex Antagonists that are believed to block NMDA receptor functioning by means of actions at glycine receptors include 7-chlorokynurenate, 7,5-dichlorokynurenate, and FG 9067 (MNQX). The latter compound, manufactured by Ferrosan, is of particular interest since it readily penetrates blood brain barriers and reportedly has potent anti-convulsant effects (Drejer et al 1989). Agents that antagonize NMDA receptor function by an action at polyamine receptors include ifenprodil and SL 82.0715, which are manufactured by Synthelabo, a company in France. It has been reported that these polyamine antagonists protect the brain against ischemic damage in an animal model of stroke (Gotti et al., 1988).

**Competitive NMDA antagonists**

Agents that block NMDA toxicity by binding directly to NMDA receptors are referred to as competitive NMDA antagonists. Such compounds include D-2-amino-5-phosphonopropanoate (D-AP5) and D-2-amino-7-phosphonoheptanoate (D-AP7). Those two compounds are of limited therapeutic utility, because they do not readily penetrate the BBB's. However, it is possible that recently developed competitive NMDA antagonists such as CGS 19755 (Boast 1988) CGP 39551, and CGP 37849 (all developed by Ciba-Geigy), 3-(2-carboxypiperazin-4-yl)-propyl-1-phosphonate (CPP) or its unsaturated analog, CPP-ene (Herrling et al 1989; developed by Sandoz), or NPG 12626 (Borosky et al 1988; developed by Nova Pharmaceutical) may penetrate BBB's in sufficient quantities to affect the CNS following systemic administration (Herrling et al

1989).

## Non-NMDA receptors and antagonists

As mentioned above, the three known types of EAA receptors are NMDA receptors, KA receptors, and QUIS receptors. KA receptors and QUIS receptors are often grouped together under the label, "non-NMDA" receptors. As used herein, any antagonist that interacts with either KA or QUIS receptors (or with both receptor types) is referred to as a non-NMDA antagonist.

Since glutamate is capable of hyperstimulating all three classes of EAA receptors, questions have arisen as to whether adequate protection from neuronal degeneration caused by conditions such as hypoxia/ischemia, persistent seizures (status epilepticus), hypoglycemia, or trauma can be achieved by the use of drugs that block only the NMDA class of EAA receptors.

In *in vitro* experiments involving pieces of chick retinas (described in the examples below and in Price 1988), the Applicant discovered that (1) blocking NMDA receptors only, using MK-801, provides only partial protection against excitotoxic lesions in retina cells deprived of oxygen and glucose, and (2) blockage of both NMDA and non-NMDA receptors offered a higher level of protection. In subsequent experiments using an *in vivo* rat retina assay (using procedures described in Mosinger and Olney 1989a and in Example 7 below), it was discovered that the use of MK-801 and CNQX in combination could provide better protection than either compound alone. That result, which extended the chick retina result to intact adult mammalian lab animals, was summarized in Mosinger and Olney 1989b, an abstract published in late 1989.

Much less is known about non-NMDA receptors than is known about NMDA receptors, for several reasons. They tend to share a higher level of cross-reactivity with each other than with NMDA receptors. Most non-NMDA antagonists do not penetrate the BBB, which makes it difficult to study non-NMDA receptors inside the BBB. Although some non-NMDA agonists such as kainic acid and domoic acid penetrate the BBB, they have such powerful effects on KA receptors that they trigger an excitotoxic glutamate cascade that excites NMDA and QUIS receptors, making it even more difficult to study KA receptors alone. In addition, non-NMDA antagonists such as kynurenic acid and 7-chlorokynurenate also have substantial affinity for NMDA receptors, which makes it difficult to study the reactions and effects of just one receptor type in either *in vitro* or *in vivo* preparations.

Despite those problems, work is actively being done to further characterize and study non-NMDA receptors and to identify compounds that selectively antagonize non-NMDA receptors. A major breakthrough was reported in late 1987, when Honore and his colleagues at Ferrosan Chemicals, in Denmark, reported that two quinoxalinedione derivatives are potent antagonists of QUIS receptors, with somewhat less but substantial affinity for KA receptors, and with very little amity for NMDA receptors (Honore et al 1987, and Honore et al 1988). Those two quinoxalinedione derivatives are 6,7-dinitro-quinoxaline-2,3-dione (DNQX also refetred to as FG 9041) and 6-cyano-7-nitro-quinoxaline-2,3-dione (CNQX also referted to as FG 9065).

Apparently, neither of those two compounds penetrates BBB's in substantial quantities. However, Honore et al subsequently reported that another quinoxalinedione derivative which acts as a non-NMDA antagonist, designated as NBQX penetrates the BBB in substantial quantities (Honore et al 1989 and Sheardown et al 1989). NBQX is 6-nitro-7-sulphamoyl-benzo(f)quinoxaline-2,3- dione, also refetred to as FG 9202.

## Neurological problems that might be aided by antagonists

EAA antagonists have been shown in animal experiments (reviewed in Olney 1989a) to reduce CNS damage caused by hypoxia/ischemia, persistent seizures (status epilepticus), hypoglycemia, trauma, thiamine deficiency, and methamphetamine poisoning (a form of neurotoxicity related to Parkinson's disease). It is possible, therefore, that EAA antagonists might be used therapeutically for neuroprotective purposes in conditions such as the above.

It is also possible that EAA antagonists might prevent brain damage associated with alcoholism. In chronic alcoholics, neuronal degeneration has been described in several regions of the brain, including periventricular and periaqueductal regions, the thalamus, the hypothalamus, and mammillary bodies. Individuals with this type of brain damage manifest a form of dementia known as Wernicke/Korsakoff syndrome, which includes severe deficits in memory and cognitive functions. It is believed that this syndrome relates to dietary deficiencies, especially thiamine (vitamin B) deficiency. It is known that people who suffer from thiamine deficiency are subject to the same pattern of brain damage and the same

dementing Wernicke/Korsakoff syndrome that is seen in alcoholism. Recently, it was demonstrated that in a rat model of thiamine deficiency, which entails disseminated brain damage distributed in a Wernicke/Korsakoff pattern, the brain damage can be markedly attenuated by pretreatment with MK-801 (Langlais et al 1988). That result suggests that this type of brain damage is mediated by an excitotoxic mechanism involving the NMDA receptor ion channel complex, and that patients with acute symptoms suggesting an impending Wernicke/Korsakoff syndrome might benefit from treatment with EAA antagonists.

It is also suspected that there may be a link between virally-induced neurodegenerative conditions and EAA-mediated excitotoxicity (Olney 1989b). When a viral infection triggers changes in neural homeostasis, endogenous excitotoxins such as glutamate and aspartate may become involved in cell death or damage. Therefore, EAA antagonists may be useful for preventing neuronal degeneration associated with viral infections that involve the CNS, such as Jacob-Creutzfeldt syndrome and encephalitis associated with herpes or measles infection.

EAA antagonists also have been shown to be useful for protecting against brain damage associated with a newly recognized type of food poisoning. In January 1988, there was an outbreak of food poisoning in Canada affecting 145 identified individuals, some of whom died and were found at autopsy to have widespread brain damage (Quilliam et al 1989). Some of the surviving victims suffered severe brain damage and became permanently demented. All of thc victims had ingested mussels from the Newfoundland region. Analysis of the mussels revealed a high concentration of domoate, a powerful convulsant which causes brain damage by inducing persistent seizure activity which releases excessive glutamate, triggering an excitotoxic cascade. It is believed that domoate poisoning may become a recurrent problem in several regions of the world. The Applicant of the subject invention has recently demonstrated that MK-801 and phencyclidine protect against domoate neurotoxicity. Therefore, EAA antagonists may serve as antidotes to prevent brain damage, dementia and/or death in domoate poisoning.

In addition to seizure-related brain damage associated with domoate poisoning, brain damage also occurs as a result of persistent seizures in patients with epilepsy. In this situation also, it is believed that excessive activation of EAA receptors by endogenous glutamate may cause or exacerbate brain damage. It is possible, therefore, that patients who come to emergency rooms in status epilepticus (a state of continuous epileptic seizure activity) might be protected from permanent brain damage by timely treatment with EAA antagonists.

However, the potential therapeutic uses of MK-801 and other NMDA antagonists must be viewed with caution, because it has recently been discovered (Olney et al 1989c) that such agents can inflict their own type of neurological damage.

## Neurotoxic side effects of non-competitive NMDA antagonists

A potentially serious side effect of MK-801, phencyclidine, and other non-competitive NMDA antagonists is that they induce a neurodegenerative reaction in the posterior cingulate and retrosplenial cerebral cortex, even when admimstered in relatively low doses (Olney et al 1989c). In a series of experiments, MK-801 and phencyclidine were given to adult rats to test for neuroprotection against seizure-related brain damage. Those agents protected neurons in certain brain regions from seizure-related damage, but they also caused a different type of neurotoxic reaction in other brain regions, the posterior cingulate and retrosplenial cerebral cortices. The neurotoxic reaction, which was observed during microscopic analysis of CNS tissue after the rats were sacrificed, consisted of the formation of vacuoles (membrane-enclosed spaces in the cytoplasm that are not present in normal cells) and the dissolution of mitochondria (energy-producing organelles inside the cells). Although these changes appeared to be reversible if the doses of MK-801 or phencyclidine were sufficiently low, it was subsequently discovered that irreversible necrosis of cingulate cortical neurons follows the administration of 5 mg/kg MK-801.

In adult rats, the $ED_{50}$ for producing vacuoles in cingulate neurons by MK-801 administration (i.e., the dosage of MK-801 which will produce vacuoles in 50% of the animals treated) is 0.18 mg/kg, administered intraperitoneally (Olney et al 1989c). Since the doses of MK-801 used in animal experiments for protecting neurons against ischemic brain damage usually are much higher, in the range of 1 to 10 mg/kg, the use of MK-801 for therapeutic neuroprotection poses a major risk of inducing serious neurotoxic side effects.

As described in Example 5, the Applicant has also discovered that MK-801 poses a risk of potentiating some types of seizures that involve the cholinergic receptor system. Although the Applicant has used MK-801 successfully to prevent brain damage associated with seizures induced by certain methods, the experiments described in sample 5 indicate that MK-801 has a potentiating effect when administered along with pilocarpine, a cholinergic agonist used in lab animals to model epileptic seizures and seizure-related

brain damage (Turski et al 1983; Clifford et al 1987). The term "potentiate" refers to the fact that the MK-801 lowered the seizure threshold and made test animals susceptible to seizures at a pilocarpine dosage that would not have caused seizures in the absence of the MK-801. This finding raises questions about whether NMDA antagonists would tend to induce seizures in humans who suffer from epilepsy. The Applicant also discovered that pilocarpine and MK-801, when admimstered together, increase the formation of vacuoles in cingulate/retrosplenial neurons. If an adult rat is treated with pilocarpine (75 mg/k ip), the $ED_{50}$ of MK-801 for producing cingulate/retrosplenial vacuoles is reduced from 0.18 mg/kg to 0.05 mg/kg.

Judging from these results, non-competitive NMDA antagonists such as MK-801 apparently can exert a beneficial neuroprotective effect in certain seizure-brain damage syndromes by blocking one of the major excitatory transmitter systems, the NMDA receptor system However, such antagonists appear to potentiate seizure activity in some cases, especially cases mediated by the cholinergic receptor system. These results imply that some kind of mechanism exists by which the cholinergic and NMDA receptor systems are linked, such that drugs affecting either system can influence neurological disorders such as seizure activity and formation of vacuoles in cingulate/retrosplenial neurons.

## Neurotoxic side effects of competitive NMDA antagonists

Recent evidence also indicates that competitive NMDA antagonists (i.e., molecules which bind directly to NMDA receptors rather than to PCP receptors) also pose a serious risk of causing neurotoxic side effects. This danger was not fully appreciated until recently, because most competitive NMDA antagonists do not penetrate blood-brain barriers (BBB's) in substantial quantities.

To overcome that factor and enable the assessment of potential neurotoxic effects of competitive NMDA antagonists, the Applicant helped develop a method for bypassing the BBB by microinjecting a competitive NMDA antagonist, D-AP5, directly into the cingulate cortical regions of rats. When D-AP5 reached those neurons, it caused vacuole formation and mitochondrial dissolution similar to the effects of non-competitive NMDA antagonists (Labruyere et al 1989, which was co-authored by the Applicant).

In a subsequent experiment, a powerful competitive NMDA antagonist with fairly low but significant BBB permeability was injected into rats intravenously, at a dosage high enough to ensure that significant quantities entered the brain. It was found to cause vacuole formation in retrosplenial/cingulate neurons, comparable to the vacuoles formed by non-competitive NMDA antagonists.

These findings indicate that any drug which blocks NMDA receptor ion channel activity by any mechanism (including competitive blocking of the NMDA receptor, non-competitive activation of the PCP receptor, or blocking of the glycine, polyamine, or other receptors that directly effect the ion channel that is regulated by the NMDA receptor) may have neurotoxic effects. An important implication is that competitive NMDA antagonists which may be able to penetrate the brain in sufficient concentration to be used as neuroprotective drugs may not provide an acceptable alternative to non-competitive NMDA antagonists such as MK-801, unless used in conjunction with a suitable neuroprotective agent.

Despite the current awareness that NMDA antagonists pose a serious risk of cingulate/retrosplenial neurotoxicity, they remain of substantial interest among researchers, because of their potential for handling crisis situations such as severe stroke, cardiac arrest, or perinatal asphyxia, which otherwise would lead to severe brain damage or death. If a person can be saved from death or severe brain damage only by using NMDA antagonists, then such treat ment may be necessary even though it entails various risks such as damage to cerebrocortical neurons, transient psychotic manifestations, and potentiation of seizure susceptibility.

## Cholinergic receptors

Cholinergic receptors are activated by acetylcholine, a relatively small molecule released by certain types of brain cells. Cholinergic receptors are divided into two main classes: muscarinic and nicotinic.

Little is known about nicotinic receptors in the CNS. They exist in the peripheral nervous system, at neuromuscular junctions, and they are presumed to exist inside the brain, but very limited progress has been made in developing agonist or antagonist molecules that can penetrate blood-brain barriers and be used to pharmacologically characterize nicotinic receptors that may exist in the brain.

Muscarinic receptors are subdivided into M1 and M2 receptors, based on the discovery that pirenzepine binds with much greater affinity to one subpopulation (M1) found primarily in the forebrain, than to a separate subpopulation (M2) that exists primarily in the hindbrain and in the peripheral nervous system.

Most anti-cholinergic molecules, although more powerful than pirenzepine in binding to cholinergic receptors, do not show as high a degree of specificity for one receptor subpopulation. Thus, the anti-cholinergic agents of primary interest herein have substantial affinity for both M1 and M2 receptors (Burke 1986; Freedman et al 1988). It is not known whether or to what extent these anti-cholinergics also interact with nicotinic receptors inside the brain, since reliable methods for identifying and characterizing nicotinic receptors in the brain have not been available.

## Anti-cholinergic agents

A group of agents classified as anti-cholinergics (i.e., they block the activation of cholinergic receptors) have been used in clinical neurology as anti-parkinsonian drugs (Goodman and Gilman 1975). These agents were recently found by the inventor to protect rats against the convulsant and brain damaging action of pilocarpine and another cholinergic neurotoxin, soman (Price et al 1989). The drugs that conferred this neuroprotective action are procyclidine, biperiden and trihexyphenidyl, which are structurally related compounds of the aryl-cyclo-alkanolamine class. These agents, especially biperiden and trihexyphenidyl, are considered cholinergic antagonists that act quite powerfully at the M1 muscarmic receptor (Freedman et al 1988), which suggests a possible explanation for their efficacy in blocking the neurotoxic actions of pilocarpine, which is primarily an M1 cholinergic agonist. These arylcyclo-alkanolamines have also been shown to have limited effectiveness as NMDA receptor antagonists (Olney et al 1987), but they are considered much more powerful as M1 cholinergic antagonists than as NMDA antagonists.

Procyclidine, which bas some degree of affinity for NMDA receptors in addition to being an anti-cholinergic agent, can be administered to adult rats at a high dose (75 mg/kg) without producing neurotoxic side effects such as cingulate/retrosplenial vacuole formation induced by other NMDA antagonists such as MK-801 or D-AP5. Procyclidine is described in US Patent 2,891,890 (Adamson 1959), and is marketed under the trade name "Kemadrin" by Burroughs-Wellcome.

Biperiden has been studied for its mood altering effects (Fleischhacker et al 1987) and for its interaction with muscarinic receptors (Syvalahti et al 1987). The hydrochloride salt of biperiden has been studied for its interaction with nicotine and oxotremorine in rat diaphragm (Das et al 1977). Biperiden is marketed under the trade name "Akineton" by Knoll.Triperiden is marketed in Europe under the trade name "Norakin" by VEB Fahlberg-List (Magdeburg, West Germany).

Trihexyphenidyl has been studied for its effects in schizophrenic patients (Hitri et al 1987) and for its effects on memory in elderly patients (McEvoy et al 1987). It is marketed under the trade name "Artane" by Lederle, and is used to reduce Parkinson symptoms in schizophrenics who are being treated with phenothiazine compounds.

Various other aryl-cycloalkyl-alkanolamine compounds have also been studied for varying purposes (e.g., US patents 4,031,245 and 3,553,225, West German Offen. No. 1,951,614, and Mann et al 1976). However, none of the research with this class of compounds involves their use for reducing the neurotoxic effects of PGP, MK-801, or other NMDA antagonists.

A number of other compounds are known to function as anti-cholinergic agents. Benztropine, sold under the trade name "Cogentin" by Merck, Sharp and Dohme, is used to reduce Parkinson symptoms in schizophrenics being treated with phenothiazine compounds (Vernier 1981). Benactyzine is used in conjunction with meprobamate in a formulation called "Deprol," sold by Wallace Laboratories (Physicians Desk Reference 1989, p. 2200). Scopolamine and atropine, both of which have been used widely in medicine for anesthesia-related purposes, have also been used as anti-Parkinsonian drugs, but they tend to cause side effects when administered at anti-Parkinson dosages, due to their interactions with cholinergic receptors in the peripheral nervous system.

In summary, accumulating evidence suggests that NMDA antagonists might be highly useful therapeutic agents in various neurological disorders. However, prior to this invention, there was no known agent or method for preventing certain deleterious side effects of those NMDA antagonists. In addition, certain tests suggest that a mixture of an NMDA antagonist and a non-NMDA antagonist, if both can penetrate the blood-brain barrier, will provide more effec tive protection against various neurotoxic disorders than either agent can provide alone.

One object of this invention is to provide pharmacological agents and methods which can be used in human or veterinary medicine to reduce the neurotoxic side effects of NMDA antagonists.

Another object of this invention is to provide a mixture of an NMDA antagonist combined with a protective agent to reduce the neurotoxic effects of the NMDA antagonist. Such mixtures can be used to prevent or minimize deleterious CNS effects associated with various diseases or traumatic processes that

can cause permanent and irreversible brain damage, such as stroke and perinatal asphyxia.

Another object of this invention is to provide a pharmacological mixture of an NMDA antagonist and a non-NMDA antagonist, for providing more complete protection against certain types of brain damage than can be achieved by NMDA antagonists alone, wherein the mixture will also contain a protective agent to reduce the neurotoxic side effects of the NMDA antagonist.

The three-component mixture described herein (an NMDA antagonist, a non-NMDA antagonist, and a protective agent to prevent neurotoxic side effects) is believed to be the most effective pharmacological agent and method discovered to date for preventing or minimizing a variety of neurotoxic syndromes that currently cause fatal or permanent and crippling brain damage to hundreds of thousands of people each year.

## SUMMARY OF THE INVENTION

This invention involves the pharmacological use of protective agents for reducing neurotoxic side effects that can be caused when NMDA antagonists such as MK-801 are used as anti-convulsants, to prevent excitotoxic damage in the central nervous system, or for anesthetic purposes in human or veterinary medicine. These protective agents include anti-cholinergic agents such as scopolamine, atropine, benztropine, trihexyphenidyl, biperiden, procyclidine, benactyzine, or diphenhydramine. When admimstered in conjunction with, or subsequent to, an NMDA antagonist, these anti-cholinergic agents greatly reduce or eliminate the deleterious side effects that can accompany NMDA antagonists (such as convulsion potentiating effects, as well as vacuole formation, mitochondrial dissolution, and possible death of cingulate/retrosplenial cerebrocortical neurons), without interfering with the useful properties of the NMDA antagonists. The protective agents described herein may also reduce the neurotoxic, psychotoxic, and/or hallucinatory side effects associated with illegal use of drugs such as phencyclidine. A suitable NMDA antagonist can be selected from the group that includes MK-801, CPP, CPP-ene, CGS 19755, CGP 39551, CGP 37849, NPC 12626, MNQX, ifenprodil, and SL 82.0715.

This invention also involves the use of an NMDA antagonist and a protective agent as described above in conjunction with a non-NMDA antagonist such as 6-nitro-7-sulfamoylbenzo(f)-quinoxaline-2,3-dione, also referred to as NBQX or as FG 9202. The NMDA and non-NMDA antagonists together provide a higher degree of protection against the primary neurotoxic damage caused by diseases or trauma than can be provided by either antagonist alone; in addition, the protective agent reduces or prevents the neurotoxic side effects that would otherside be caused by the NMDA antagonist.

The agents described herein, when used in combination, offer greater protection against several major types of brain damage than can be obtained by any agents, mixtures, or methods previously described for this purpose.

## DESCRIPTION OF THE PREFERRED EMBODIMENTS

This invention involves a pharmacological agent and method for protecting against excitotoxic damage to neurons in a mammalian central nervous system, which can occur in conditions including hypoxia/ischemia, stroke, epilepsy, hypoglycemia, brain trauma, or certain types of food poisoning. This agent comprises a mixture of an NMDA antagonist and an anti-cholinergic protective agent, both of which penetrate blood-brain barriers (BBB's) to an extent that allows them to inhibit the functioning of NMDA receptors and acetylcholine receptors on neurons inside the brain.

Suitable NMDA antagonists can function through any mechanism which effectively inhibits the functioning of the NMDA receptor or its associated ion channel. For example, suitable NMDA antagonists might comprise non-competitive NMDA antagonists such as MK-801, phencyclidine, ketamine, and tiletamine, which activate the phencyclidine receptor. Alternatively, competitive NMDA antagonists that block one or more portions of the NMDA receptor itself, such as CGS 19755, CGP 39551, CGP 37849, CPP, CPP-ene, or NPC 12626, can be used, provided that the dosages required for penetration of the BBB in quantities adequate to inhibit NMDA receptors do not cause unacceptable levels of adverse side effects. As another alternative, NMDA antagonists (such as ifenprodil, SL 82.0715, or MNQX) that block a glycine or polyamine receptor which functions as a part of an NMDA receptor complex may also be suitable for the purposes of this invention; this can be confirmed using routine experimentation with lab animals, as described below.

This invention involves the use of anti-cholinergic protective agents, in conjunction with the NMDA antagonists discussed above, to reduce or prevent the neurotoxic effects of the NMDA antagonists. These protective compounds are classified as anti-cholinergic agents, since they block the triggering effects of acetylcholine molecules at cholinergic receptors. Prior to this invention, it was not realized that anti-cholinergic agents could prevent neurotoxic ef fects caused by NMDA antagonists, since NMDA antagonists affect glutamate receptors, which are entirely different and distinct from cholinergic receptors.

As used herein, the terms "neurotoxic effects" and "deleterious neurological effects" caused by NMDA antagonists refer to any of the following: (1) the formation of observable vacuoles in any type of CNS cell, if comparable vacuoles of similar number, size, or type are not detected in neurons of the same type in healthy, untreated animals; (2) the causation of damage to, dissolution of, or other significant alterations in substantial numbers of mitochondria in CNS cells; (3) the death of, or necrotic signs in, significant numbers of cerebrocortical neurons if attributable to administration of an NMDA antagonist; (4) the generation of psychotoxic or hallucinatory effects, such as the psychotomimetic effects of PCP, or the "emergence reaction" suffered by some patients who are anesthetized by ketamine; or, (5) convulsion potentiating effects.

The anti-cholinergic agents which have been tested thus far to determine whether they can prevent neuronal vacuole formation caused by NMDA antagonists such as MK-801 and PGP include the following compounds, which are listed in decreasing order of potency, measured by their ability to prevent vacuole formation by MK-801 administered at 0.4 mg/kg (0.4 milligrams of MK-801 per kilogram of animal body weight, as described in Example 1):

1. Scopolamine provides the highest degree of protection discovered to date. It was completely effective on all animals tested to date at 0.25 mg/kg.
2. Benztropine provided complete protection for all animals tested to date at 2 mg/g.
3. Trihexyphenidyl and atropine protected all animals tested to date when administered at 5 mg/kg.
4. Biperiden, procyclidine, and benactyzine protected all animals at 10 mg/kg.
5. Diphenhydramine, which is usually classed as an H1 histamine antagonist but which also has anti-cholinergic activity, protected all animals at 25 mg/kg.

Additional information is contained in Table 1. The test data are based on limited numbers of tests done to date, using the procedures described in the Examples. As will be recognized by those skilled in the art, these data will need to be confirmed and carefully evaluated by more extensive testing; however, the results obtained thus far are quite clear and highly significant.

A second key finding is that the anti-cholinergic agents listed above and in Table 1, at doses insufficient to totally prevent vacuole formation, substantially decreased both the number and the size of the cingulate/retrosplenial vacuoles that were caused by the MK-801. This result is not reflected in the tables, which indicate only the presence or absence of any detectable vacuoles. Clearly, a substantial reduction in the number and/or the size of vacuoles indicates that the neurotoxic properties or activities that are manifested by vacuole formation are being mitigated by the anti-cholinergic agents.

The anti-cholinergic compounds tested to date are all commercially available; in addition, methods of synthesis of these compounds are known to those skilled in the art. For example, synthesis of procyclidine and its salts are shown in US patents 2,891,890 and 2,826,590. Synthesis of trihexyphenidyl hydrochloride is described in US patent 2,682,543. Synthesis of biperiden is described in US patent 2,789,110.

Some anti-cholinergic agents (such as dicyclomine and pirenzepine) may be of limited value for protecting against NMDA antagonist neurotoxicity, since they bind only weakly to cholinergic receptors in the brain. It should be noted that dicyclomine and pirenzepine are often referred to in the literature as "highly selective" M1 antagonists. This can be confusing, since their affinity for M1 receptors is relatively weak compared to other M1 antagonists such as scopolamine. The "highly selective" M1 attribute derives from the fact that they have extremely weak affinity for M2 receptors, as indicated in Table 2, which is derived from Freedman 1988 at page 136. The $K_{app}$ values provided by Freedman et al are, in effect, dissociation values; a high $K_{app}$ value indicates weak binding. Therefore, the value $1/K_{app}$ provides an indicator of affinity. As can be seen from Table 2, although dicyclomine and pirenzpine have high M1/M2 ratios, their affinity for M1 receptors is only a small fraction of the affinity of other compounds such as scopolamine.

In addition, preliminary experiments indicate that N-methylscopolamine is of virtually no value for use as described herein, even though it is a potent anti-cholinergic agent. This was expected, since N-methylscopolamine does not penetrate the blood-brain barrier in substantial quantities.

The data in Tables 1 and 2 suggest that the blocking of M1 receptors is heavily involved in the neuroprotective action of anti-cholinergic agents. This follows from the data indicating that atropine offered a lower degree of protection than scopolamine. However, all of the anti-cholinergics tested to date have a

substantial degree of affinity for both M1 and M2 receptors.

As mentioned previously, it is not known whether or to what extent any anti-cholinergic agents bind to nicotinic receptors inside the CNS, because of the lack of appropriate methods for studying nicotinic receptors within the CNS. As the state of knowledge regarding nicotinic receptors advances, any anti-cholinergic agents which pass through the blood-brain barrier and which are reliably identified as antagonists of CNS nicotinic receptors can be screened for protective activity against the neurotoxic effects of NMDA antagonists such as MK-801, using the procedures described herein. Despite the current limited knowledge regarding nicotinic receptors, every anti-cholinergic agent tested to date which can penetrate the blood-brain barrier has been demonstrated to be effective in reducing the neurotoxic side effects of MK-801.

One of the anti-cholinergic compounds (biperiden) has also been shown to be effective in preventing vacuole formation by phencyclidine (PCP), as described in Example 3. Since PCP and MK-801 both bind to and activate PCP receptors in the CNS, it is believed that any anti-cholinergic compounds which provide effective protection against the side effects of MK-801 will also protect against PCP neurotoxicity, and therefore could be used to counteract the permanent damage and possibly the psychotic symptoms of PCP ("angel dust") when abused by illegal users.

The anti-cholinergic compounds discussed herein have also been tested to ensure that they do not interfere with the desirable neuroprotective properties of MK-801, an agent that shows great promise in blocking excitotoxic damage involving glutamate receptors. As described in Example 4, the results are quite favorable; the anti-cholinergic agents tested to date apparently do not interfere with the beneficial effects of MK-801.

Another set of experiments, described in Example 5, indicate that NMDA antagonists may have convulsion-potentiating effects when cholinergic receptors are being excessively stimulated. The NMDA antagonist used (MK-801) lowered the seizure threshold and made animals more susceptible to seizures, when the animals were treated with a cholinergic agonist (pilocarpine) at a dosage that would not have caused seizures in the absence of the NMDA antagonist. This finding raises questions about whether NMDA antagonists would also tend to induce seizures in humans who suffer from epilepsy. However, it was also discovered that the administration of biperiden, an anti-cholinergic, along with MK-801 and pilocarpine, protected the animals against any seizure activity, brain damage, and vacuole formation in cerebrocortical neurons. This result indicates that the vacuole-inducing properties and the convulsion potentiating properties of an NMDA antagonist such as MK-801 can be prevented by an anti-cholinergic agent.

One preferred embodiment of the subject invention comprises a mixture of (1) an NMDA antagonist such as MK-801, and (2) an anti-cholinergic protective agent. The NMDA antagonist can be used for beneficial purposes such as human or veterinary anesthesia or to protect against excitotoxic or neurodegenerative processes associated with various conditions described above. The anti-cholinergic agent will prevent or minimize deleterious side effects (such as convulsions, hallucinations, or pathological changes or necrosis of cerebrocortical neurons) that might otherwise be caused by the NMDA antagonist. By reducing the deleterious side effects of the NMDA antagonist without interfering with its useful activity, the anti-cholinergic agent can render the mixture a more safe and effective formulation.

**Non-NMDA Antagonists**

An alternate preffered embodiment of the subject invention involves the use of a mixture of three different components: (1) an NMDA antagonist, as described above; (2) an anti- cholinergic protective agent, as described above; and (3) a non-NMDA antagonist.

A non-NMDA antagonist suitable for use as described herein comprises NBQX, a common name for 6-nitro-7-sulphamoyl-benzo(f)quinoxaline-2,3-dione), a quinoxalinedione derivative. Sheardown et al 1989 reports that NBQX exerts a neuroprotective effect against surgically-induced ischemia inside the brain; this indicates that NBQX permeates the BBB.

NBQX is the first quinoxalinedione derivative that reportedly permeates the BBB; however, quinoxalinedione derivatives are of great interest in neuroscience, because of their ability to selectively block non-NMDA receptors, and it is highly likely that other quinoxalinedione analogs and derivatives will be discovered in the near future which also permeate BBB's. Any such quinoxalinedione derivative is likely to be suitable for the purposes of this invention, if it has two characteristics: (1) it must antagonize at least one type of non-NMDA receptor, and (2) it must penetrate BBB's in quantities sufficient to inhibit the functioning of non-NMDA receptors.

The first criterion can be evaluated using *in vitro* tests such as the chick retina test described below, or

by using the in vivo rat retina assay developed by the Applicant, also described below. Any quinoxalinedione derivative or other compound which blocks KA and/or QUIS receptors can be further evaluated to determine whether it permeates BBB's, using any of several *in vivo* tests, including the following:

(1) Ischemia in the brain can be surgically induced by temporarily blocking the major arteries that supply blood to the brain. The animals are subsequently sacrificed, and the brains are histologically examined to assess the extent of any neurotoxic lesions that are characteristic of ischemia (described in more detail below). If a prior-administered drug reduces the severity of such lesions in the brain, this indicates that the drug penetrated the BBB in sufficient quantities to exert a neuroprotective effect. A surgical procedure is commonly used (e.g., Kirino et al 1984) which involves clamping the common carotid arteries in Mongolian gerbils. Gerbils are used, since they have a vascular arrangement that causes carotid occlusion to induce cerebral ischemia, whereas rats have vascular arrangements that shunt cerebral blood flow through other arteries. The carotids of the gerbils are clamped for a limited time (usually about five minutes) then they are unclamped and the animals are allowed to fully recover, usually for several days, before they are sacrificed and the CA1 cells of the hippocampus are examined. If desired, the vertebral arteries can also be clamped or cauterized to induce a more severe state of ischemia, ischemia affecting additional brain regions, or ischemia in other types of lab animals (Pulsinelli et al 1979), or the middle cerebral artery can be blocked to create localized ischemia (Tamura et al 1981).

(2) Experiments can be performed using radioactive isotopes of a quinoxalinedione derivative that is of interest (i.e., the derivative can be synthesized using isotopes of hydrogen, carbon, oxygen, etc). The radiolabelled compound is injected into the animal; the animal is subsequently sacrificed, and its brain is analyzed to determine whether the isotope entered the brain in the injected form.

(3) Glutamate, aspartate, or other molecules which excite one or more classes of EAA receptors (such as kainic acid or quisqualate) can be microinjected into a specific region of an animal brain. The animal is subsequently sacrificed, and that region of the brain is analyzed to assess the severity of the excitotoxic lesions induced by the agonist. If a previously-injected antagonist can reduce those lesions or prevent their formation, that indicates that the antagonist penetrated the BBB.

(4) Kainic acid (KA), if injected into an animal at quantities of 12 mg/kg or greater, will penetrate the BBB and cause a seizure by excessively stimulating the KA-class of EAA receptors. If a specific quinoxalinedione derivative has substantial affinity for KA receptors (regardless of whether it also has any affinity for QUIS receptors), the ability of that derivative to permeate the BBB can be assessed by determining whether it is capable of blocking KA-induced seizures.

Although some of these tests are labor-intensive, all can be carried out using routine experimentation.

It has been known for some time that NMDA antagonists provide partial protection against certain types of excitotoxic CNS damage caused by problems such as hypoxia/ischemia. The discovery that a combination of NMDA and non-NMDA antagonists provide more effective protection against hypoxic/ischemic CNS damage than either class of agent can provide by itself arose out of two types of specialized assays that were developed or co-developed by the Applicant, both of which involve neurons in the retina (i.e., the light-sensitive nerve cells at the back of the eye), which are regarded as part of the CNS. Since many retinal neurons undergo a toxic reaction when exposed to NMDA, KA, or QUIS (agonists that selectively activate the three main classes of EAA receptors), it is believed that those neurons contain all three types of EAA receptors.

In the experiments described in Example 6, the Applicant and his colleagues modified a chick embryo retina assay (originally described in Reif-Lehrer et al 1975) to make it suitable for studying ischemic neuronal degeneration. Briefly, chick embryos were removed from their eggshells, and pieces of retinal tissue were isolated and incubated in tissue culture dishes containing nutrient solutions. To simulate hypoxia-ischemia, glucose and dissolved oxygen were eliminated from the incubation medium, and the tissue sections were incubated in an oxygen-free atmosphere under a blanket of inert gas. After 30 min of incubation under those conditions, the retinas displayed neurodegenerative lesions identical to the lesions observed when retinas are incubated in medium containing a toxic concentration of glutamate.

Including either an NMDA antagonist (MK-801, 0.1 to 100 micromolar (uM)) or a non-NMDA antagonist (CNQX 10 to 100 uM) in the incubation medium provided only partial protection against neuronal degeneration caused by oxygen and glucose deprivation. However, when both types of antagonists (MK-801, 0.2 uM and CNQX, 50 uM) were administered together, they completely prevented the degenerative reaction. These results are shown in Table 3.

Substantial doubts existed as to whether the results of the chick embryo retina assay were applicable to an understanding of human neurological disorders such as cerebral ischemia caused by stroke. The reasons for uncertainty include the following: (1) the chick retina assay is only a simulated model of

ischemia, which relies on small pieces of tissue cultured *in vitro* rather than an *in vivo* system using an intact animal; (2) the chick assays uses embryonic rather than adult CNS tissue; and, (3) the chick assay uses an avian species rather than a mammal.

To resolve those areas of uncertainty, an entirely different assay was developed for studying CNS ischemic neuronal degeneration. This assay, which involves an *in vivo* test using induced ischemia in intact adult mammals, is described in Examples 7 and 8.

Briefly, a specific type of dye (rose bengal dye) was selected because it undergoes a chemical change and releases an oxygen radical (i.e., a singlet oxygen atom with an unpaired electron, which is highly reactive) when exposed to bright light having a certain wavelength. This dye is injected into the tail vein of an adult rat, and is allowed to circulate in the rat until it enters the retina. The rat's eye is dilated, and a bright light having the proper wavelength is shone into the eye, causing the dye in the retinal blood vessels to release oxygen radicals, which activate a blood clotting mechanism that causes a blood clot to form rapidly in the illuminated blood vessels of the retina. Within a few minutes after the light exposure, the arterial circulation to the retina is totally occluded. Therefore, this method offers a non-invasive way to induce ischemia *in vivo* in the adult mammalian CNS, and provides a very useful model for studying cerebral thrombosis (usually called stroke), a condition that occurs m humans hundreds of thousands of times per year.

This is a particularly suitable model as it is not as labor intensive nor as invasive as other experimental models, and it has the special advantage of allowing an experimental drug to be delivered to the ischemic CNS tissue. The majority of *in vivo* research on cerebral ischemia uses surgically invasive methods, which entail tying off or cauterizing major blood vessels to portions of the brain. If such steps are taken to generate ischemic conditions in a certain region of the brain, one must then face the dilemma that one cannot deliver the experimental drug to the vulnerable tissue site, because the blood vessels to that region have been tied off or cauterized. In the photothrombosis retina model, the blood vessels are occluded but the neuroprotective drug can be injected into the vitreous humour of the eye, from which it dif fuses freely into all parts of the retina.

It should also be noted that some models of ischemic damage in the CNS generate "global ischemia," while others generate "focal ischemia." Focal ischemia can be induced in a selected portion of the brain by closing off an artery which supplies blood only to that region of the brain, while global ischemia, which affects the entire brain, can be induced (at least in gerbils) by closing the carotids. It is often difficult to evaluate and compare articles which report that certain agents are effective in preventing ischemia, since they sometimes involve limited and non-severe models of ischemia. The adult rat retina photothrombosis model induces a very severe form of ischemia which simultaneously generates a combination of both focal and global ischemia; the blood supply is shut off focally at a central point where the arteries enter the retina, and this globally deprives the entire retina of its blood supply. It thereby offers a challenging model for evaluating potential protective agents.

As described in Example 7, and as briefly summarized in Mosinger and Olney 1989b, rat retina photothrombosis tests indicated that mixtures of MK-801 and CNQX could reduce ischemic damage more effectively than either agent acting along. However, since CNQX does not penetrate the blood-brain barrier, those results do not offer any effective method of preventing ischemic damage inside those portions of the CNS that are protected by the BBB (which, of course, includes nearly all of the brain).

As described in Example 8, a subsequent set of photothrombosis tests were caried out using NBQX a quinoxalinedione which does penetrate the BBB, and MK-801. Those tests indicated that NBQX when administered by itself, reduced the level of damage by about 30%, while MK-801, when administered by itself, reduced the level of damage by about 40%. By contrast, when NBQX and MK-801 were administered in conjunction with each other, they reduced the level of damage by more than 80%.

These results clearly indicate that a mixture of an NMDA antagonist which penetrates the blood brain barrier, and a non-NMDA antagonist which also penetrates the blood-brain barrier, can reduce neurotoxic ischemic damage in CNS neurons by a much greater level than either type of antagonist acting alone. These two classes of compounds, when used in combination, provide a higher degree of protection against severe ischemic damage than any other pharmacological agent or mixture previously reported.

There is a sound basis for believing that this finding holds for neurons not just in the retina, but also in the brain and spinal cord. When any of the three prototypic EAA receptor agonists (NMDA, KA, or QUIS) is injected into any region of a mammalian CNS, it causes excitotoxic degeneration of most of the neurons present in the injected region. This indicates that most of the neurons in the CNS have all three types of EAA receptors on their surfaces. It is known that glutamate (Glu) is a universal agonist that can act at any of the three EAA receptor subtypes to cause neuronal degeneration. It follows that when endogenous Glu is released in excitotoxic concentrations into the extracellular fluid surrounding the neurons, which occurs

under pathological conditions such as stroke, neurons that have all three receptor types on their surfaces will be destroyed unless all three receptor types are blocked. Blockage of only one receptor type will not be sufficient to protect such neurons, since Glu can still destroy the neuron by excitotoxic action at the other receptor types.

Included within the family of agents useful for the purposes described herein are any tautomeric or isomeric forms, analogs, or salts of the compounds described herein, provided that such tautomers, isomers, analogs, and salts are both (1) functionally effective in the manners described herein, and (2) physiologically acceptable without causing intolerable levels of side effects. The ability of any tautomer, isomer, analog, or salt to antagonize an NMDA receptor, a non-NMDA receptor, or a cholinergic receptor can be tested using various methods known to those skilled in the art, such as competitive binding assays using radioactive isotopes of prototypic agonists or antagonists, as described in, e.g., Freedman et al 1988 or Burke 1986. The term "pharmaceutically-acceptable salts" embraces alkali metal salts as well as addition salts of free acids or free bases. The nature of the salt is not critical, provided that it is non-toxic and does not substantially interfere with the desired activity. Examples of acids which may be employed to form pharmaceutically acceptable acid addition salts include inorganic acids such as hydrochloric acid, sulphuric acid and phosphoric acid, and organic acids such as maleic acid, succinic acid and citric acid. Other salts include salts with alkali metals or alkaline earth metals, such as sodium, potassium, calcium and magnesium. All of these salts may be prepared by conventional means.

For the purposes of this invention, it does not matter whether an antagonist that acts at a specific type of receptor has any other pharmaceutical effect, so long as it is capable of blocking at least one type of receptor as described herein. For example, diphenhydramine is an anti-cholinergic agent even though it also has the characteristic of being an anti-histamine. Similarly, a compound is regarded as an anti-cholinergic agent if it blocks at least one type of cholinergic receptor (such as the M1 muscarinic receptor), regardless of whether it also blocks other types of muscarinic or nicotinic cholinergic receptors.

Administration of the compounds of this invention to humans can be by any technique capable of introducing the compounds into the bloodstream of a human patient, including oral administration or intravenous, intramuscular and subcutaneous injections. The active compound is usually administered in a pharmaceutical formulation such as in a liquid carrier for injection, or in capsule form for ingestion, although in some acute-care situations any or all of these agents might be injected without a diluting agent. Such formulations may comprise a mixture of more than one anti-cholinergic compounds together with one or more phar maceutically acceptable carriers or diluents. Other therapeutic agents may also be present in the formulation. Delivery of the active compound in such formulations may be by various routes including oral, nasal, topical, buccal and sublingual, or by parenteral administration such as subcutaneous, intramuscular, intravenous, or intradermal routes.

The various components of the mixtures described herein can be mixed together and administered simultaneously (in a single injection, infusion, etc.) if desired. Alternately, they can be administered separately (sequentially, or via different routes of administration), so long as their therapeutic effects overlap in time. In addition, the agents of this invention may be administered in conjunction with one or more thrombolytic agents (i.e., agents which help dissolve blood clots, such as streptokinase or tissue plasminogen activator) if desired.

## EXAMPLES

### EXAMPLE 1: TESTING ANTI-CHOLINERGICS AT VARYING DOSAGES

Adult female Sprague Dawley rats, approximately 4 months old, were used in these experiments. It was previously established (Olney et al 1989c) that the $ED_{50}$ for producing vacuoles in female Sprague Dawley rats is 0.18 mg/kg of MK-801 when administered subcutaneously (sc). In those earlier experiments, MK-801 produced the vacuole effect in 75% of the animals at 0.2 mg/kg sc, and in 100% at doses of 0.3 and 0.4 mg/kg sc. In the present experiment, MK-801 was administered at a dose of 0.4 mg/kg sc, a dosage more than high enough to produce vacuoles in all animals.

Ten minutes after the MK-801 was administered, the rats received an intraperitoneal (ip) injection of scopolamine, benztropine, trihexyphenidyl, atropine, biperiden, procyclidine, benactyzine, or diphenhydramine, at a range of different doses shown in Table 1. Four animals were used in each treatment group, with a concurrent control group (MK-801 alone) for each test compound.

It was previously established (Olney et al 1989c) that the vacuole effect becomes clearly observable within 4 hours following MK-801 treatment. Therefore, the rats were anesthetized and sacrificed by perfusion fixation of the CNS at 4 hours following MK-801 treatment, using a mixture of glutaraldehyde and paraformaldehyde injected into the left cardiac ventricle. Their brains were processed by methods permitting histopathological evaluation by both light and electron microscopy, as described in Olney 1971. The tissue samples were evaluated using numerical codes, by an experienced histopathologist who had no knowledge of the treatment conditions for any given tissue sample.

In all control rats (32 of 32), a severe vacuole reaction in cingulate/retrosplenial cerebrocortical neurons was observed. As indicated in Table 1, all of the anti-cholinergic agents prevented vacuole formation in a dose-related manner. All except diphenhydramine provided total protection at doses above 5 mg/kg, and atropine, trihexyphenidyl, benztropine, and scopolamine gave complete protection at doses above 2,2,1, and 0.1 mg/kg, respectively. When vacuoles were present in rats treated with doses below the thresholds for complete protection, the number and size of the vacuoles usually appeared smaller than in the control rats. Thus, the degree of protection conferred is conservatively stated when described in terms of the number of brains with vacuoles.

**EXAMPLE 2: TESTING MK-801 AT VARYING DOSAGES**

The lowest dose of biperiden (10 mg/kg) that provided complete protection against MK-801 vacuole formation in Example 1 was administered to rats that received MK-801 on an increasing dose schedule (1, 2.5 and 5 mg/kg). It was found that the same low dose of biperiden (10 mg/kg) that had protected against 0.4 mg/kg MK-801 in Example #1 also protected completely against doses of MK-801 up to and including 5 mg/kg. In all experiments, the biperiden was well-tolerated by the rats. The *Physicians Desk Reference* indicates that the $LD_{50}$ for biperiden in adult rats is 270 mg/kg, so the margin of safety between effective and toxic doses is quite favorable.

**EXAMPLE 3: PCP TESTING**

In order to determine whether anti-cholinergic agents protect against the vacuole-forming neurotoxicity of NMDA antagonists other than MK-801, female adult Sprague Dawley rats were treated with PCP (10 mg/kg sc). Ten minutes later, they were treated with biperiden at doses of 10,5, and 1 mg/kg ip. A dose-related protective effect was demonstrated. PCP caused a severe vacuole reaction in all control rats (4/4), and in 0/4, 0/4, and 1/4 rats treated with biperiden at 10,5, and 1 mg/kg respectively.

**EXAMPLE 4: NON-INTERFERENCE WITH DESIRED MK-801 EFFECTS, USING NMDA AT EXCITOTOXIC LEVELS**

To determine whether anti-cholinergics interfere with the ability of MK-801 to protect neurons against the excitotoxic action of NMDA, an ex vivo chick embryo retina assay was used. This preparation has been found useful for studying the excitotoxic effects of NMDA on CNS neurons and for evaluating the ability of antagonists to protect against NMDA excitotoxicity (Olney 1989a). In these experiments, 18 segments of chick retina were incubated, each in a separate well, in medium containing NMDA at a concentration of 80 micromolar (uM), which previously had been determined to produce a fully developed excitotoxic lesion in 30 minutes. MK-801 was added to 12 wells at 0.2 uM, a threshold concentration known to consistently block the neurotoxic action of NMDA; in 6 of those wells, both MK-801 (0.2 uM) and biperiden (50 uM) were added.

In the six wells containing NMDA but no MK-801, a full retinal lesion was present within 30 minutes. In all twelve wells that contained both NMDA and MK-801, the MK-801 blocked the neurotoxic action of the NMDA, either in the presence of biperiden (6/6) or in absence of biperiden (6/6). Therefore, even when biperiden was present in CNS tissue at a concentration 250 times higher than the concentration of MK-801, it did not prevent the MK-801 from exerting its neuroprotective action against the excitotoxic processes mediated by NMDA triggering of NMDA receptors.

In Example 4, it required a dose of only 10 mg/kg biperiden to completely block the neurotoxic effects of 5 mg/kg MK-801 on cingulate/retrosplenial cortical neurons. Thus, when BPN and MK-801 are present in a 2:1 ratio, biperiden protects against MK-801's neurotoxic side effects, but when present in a 250:1 ratio,

biperiden does not interfere with MK-801's ability to potect against NMDA receptor-mediated neurodegenerative processes.

**EXAMPLE 5: DEMONSTRATION OF THE CONVULSION-POTENTIATING EFFECTS OF MK-801, AND PROTECTION USING BIPERIDEN**

Pilocarpine is a cholinergic agonist which causes seizures and seizure-related brain damage. It is used in research pertaining to epilepsy, since it is believed that the receptor mediated processes involved in pilocarpine-induced seizures are similar in some respects to the mechanisms of epileptic seizures. Although the pilocarpine dosage that induces seizures and seizure-related brain damage varies between individual rats, it is usually in the range of about 380-400 mg/kg SC.

The Applicant previously established that MK-801 can reduce or eliminate seizures and seizure-related brain damage caused by various methods (Clifford et al 1989). However, when the Applicant tried to use MK-801 to reduce seizures induced by pilocarpine, he discovered that the combination of MK-801 and pilocarpine resulted in a severe seizure-brain damage syndrome at relatively low doses (50 mg/kg SC pilocarpine, injected 10 minutes after 1 mg/kg SC MK-801). At those doses, neither agent by itself can cause seizures or seizure-related brain damage. The MK-801 therefore potentiates the effects of pilocarpine, i.e., it lowers the seizure threshold and makes the animal more susceptible to a seizure. These observations--that MK-801 protects against some types of seizure-brain damage syndrome but potentiates other such syndromes--suggest that MK-801 might have unpredictable and in some cases adverse effects in humans who are subject to epileptic seizures, and in humans who are being treated with various neuropharmacological drugs.

Adult rats were also treated with both MK-801 (1 mg/kg sc) and biperiden (10 mg/kg ip); ten minutes later, they were injected with pilocarpine (50 mpg sc). The additional presence of the biperiden prevented all neurotoxic manifestations, including seizure activity, the widespread brain damage that would otherwise have been caused by the pilocarpine and MK-801, and the formation of vacuoles in cingulate/retrosplenial neurons.

The observation that MK-801 potentiates the convulsant effects of pilocarpine adds further evidence that a linking mechanism connects the excitotoxic processes that involve cholinergic receptors and NMDA receptors. The discovery that a cholinergic antagonist can block the convulsion potentiating effects of MK-801 further confirms that link, and indicates that by combining an anti-cholinergic agent with an NMDA antagonist such as MK-801, it is possible to utilize the anti-convulsive properties of both agents while eliminating the convulsion potentiating effects of the NMDA antagonist.

**EXAMPLE 6: CHICK RETINA ASSAYS**

Reif-Lehrer et al 1975 initially described an assay in which pieces of embryonic chicken retina were isolated and cultured *in vitro* in various nutrient media. That assay was subsequently modified and adapted by the Applicant and his coworkers in a manner that made the assay suitable for studying ischemic neuronal degeneration Ischemia (lack of blood supply) was simulated by eliminating the two major constituents of blood (glucose and oxygen) that CNS tissue depends on for energy. This was done by removing glucose from the incubation medium, and conducting the experiments in an oxygen-free atmosphere, under a blanket of inert gas such as nitrogen After 30 min of incubation under oxygen/glucose deprived conditions, chick retina tissue displays a neurodegenerative reaction identical to that observed when the retina is incubated in medium containing a toxic concentration of Glu.

Using that assay, the Applicant and his coworkers tested the excitotoxic effects of EAA agonists and evaluated the ability of various EAA antagonists to protect retinal neurons against those excitotoxic effects. The results are provided in Table 3.

As reported in Olney et al 1987 and 1988, NMDA antagonists such as MK-801 and D-APS protect against NMDA neurotoxicity, but do not provide any significant protection against KA or Quis neurotoxicity, and do not protect against glutamate toxicity. Olney et al 1987 also reported that thiamylal, a thiobarbiturate with weak broad-spectrum EAA antagonist activity, could prevent Glu neurotoxicity. Price et al 1988 (which was co-authored by the Applicant, and which was only an abstract) subsequently reported that a non-NMDA antagonist, CNQX, protected against the neurotoxicity of quisqualate, an agonist that selectively excites the QUIS receptors. Price et al 1988 also asserted that CNQX at 50 micromolar concentration prevented NMDA toxicity in the chick retina assay; however, that was subsequently discovered to be erroneous, and it is

currently believed that CNQX offers little if any protection against NMDA toxicity. Price et al also stated that a mixture of 500 nm MK-801 and 100 um CNQX could prevent ischemic damage in oxygen-deprived chick retinas.

It was recognized that evidence generated in the chick embryo retina assays might not be directly applicable to an understanding of mechanisms operative in human neurological disorders such as cerebral ischemia (stroke), for several reasons. It is only a chemically-simulated model of ischemia, using small pieces of tissue *in vitro* rather than intact organs *in vivo*. In addition, it uses embryonic tissue rather than adult tissue, and it uses an avian rather than mammalian specie.

To overcome those shortcomings and uncertainties, a different model system was developed for studying CNS ischemic neuronal degeneration, as described in Example 7.

**EXAMPLE 7: PHOTOTHROMBOTIC ISCHEMIA IN ADULT RAT RETINAS.**

A photothrombosis model was developed for studying ischemic neuronal degeneration. It uses a photosensitive dye, rose bengal dye, which releases singlet oxygen (a highly reactive atom with an unpaired electron) when bright light having a wavelength of 560 nm is shone upon it. If the dye is in the blood stream, the release of singlet oxygen triggers a blood clotting mechanism that causes a clot to form rapidly in the illuminated blood vessel. This method is described in detail in Mosinger and Olney 1989a. Briefly, adult rats (female Sprague Dawley rats, 200-300 g) were anesthetized with halothane and placed in a stereotaxic holder. Their pupils were dilated with an eye-drop mixture of phenylephrine hydrochloride and tropicamide. A Hamilton microsyringe guided by a micromanipulator was used to inject 7 ul of saline into the vitreous of the right (control) eye, and 7 ul of solution containing CNQX (60 nmol) and/or MK-801 (30 or 160 nmols) into the left (experimental) eye. Plastic contact lenses with a drop of Goniosol were then placed on the corneas of both eyes.

After fifteen minutes, rose bengal dye (40 mg/kg) was injected intravenously through the tail vein. It circulated through the heart and throughout the arterial circulation, including the retina. Both eyes were immediately exposed to 7 min of intense light from slide lantern projectors fitted with a 550 nm filter. The light caused the rose bengal dye in the illuminated blood vessels of the retina to release singlet oxygen, which triggered thrombosis (the formation of blood clots). Within a few minutes the arterial circulation to the retina was totally occluded, leading to ischemia and hypoxia in the retina. The rats were killed 1 hr later with an overdose of chloral hydrate. The eyes were removed, and the anterior portion (lens, cornea and iris) was excised and discarded. The remaining eyecup was immersed in glutaraldehyde/paraformaldehyde fixative and processed for light microscopy, as described below.

The experimental design (injecting saline into one eye to serve as a control, while injecting CNQX and/or MK-801 into the other eye) allowed one eye of each animal to serve as an internal control for each experiment. If the photothrombosis reaction did not work because the dye was not successfully administered, it would be detected as a photothrombosis failure (no histological damage) in the control eye, and that particular experiment could be voided. In practice, photothrombosis failure rarely occurred; one hour after the occlusion, the control eyes of the test animals quite consistently showed signs of a severe cytopathological reaction which closely resembles the excitotoxic type of reaction that occurs in the retina if a toxic dose of Glu is injected into the vitreous. This type of reaction, whether caused by photothrombosis or by injection of Glu into the vitreous, consists of severe swelling of neuronal dendrites and cell bodies with the nuclei of the affected neurons becoming shrunken and dark. These changes occur in three types of retinal neurons referred to as ganglion cell neurons, amacrine neurons, and bipolar neurons. When these neurons show such changes, especially swelling of the cell body and darkening of the nucleus, it is generally considered a sign of irreversible degeneration leading inexorably to cell death. In very early stages of such a reaction, the first pathological change that can be detected is mild swelling of dendrites. If the reaction is arrested at this stage, it is believed that the changes are reversible and the neuron has been saved from cell death.

CNQX is only sparingly soluble in aqueous medium, and the volume of solution that could be injected into the vitreous of the eye without creating abnormal pressure conditions was limited to approximately 7 ul. Therefore, the highest dose of CNQX that could be administered was 60 nanomoles (nmol, which is $10^{-9}$ moles, only 1/1000th of a micromole). While this dose of CNQX by itself appears to have conferred some protection, the difference in scores between the control and experimental eyes for this group was not statistically significant. Moreover, the range of variation among the experimental eyes treated with CNQX but not MK-801 was considerable, suggesting that any protective effect that may have been operative was highly inconsistent. MK-801 is quite soluble in aqueous medium and it is such a powerful NMDA antagonist

that it does not require high doses to block NMDA receptors. In fact, analysis of the data in table 1 suggests that both of the doses tested (30 and 160 nmol) were high enough to completely block all NMDA receptors. This conclusion follows from the fact that both of the doses tested conferred a similar degree of protection, as would be expected if both blocked the same number of NMDA receptors. The fact that the lower dose provided the same modest degree of protection as the higher dose signifies that this is a ceiling effect constituting the maximum amount of protection that can be obtained by using an NMDA antagonist alone.

To evaluate the neuroprotective effects achieved by the EAA antagonists, light microscopic sections 1 um thick displaying the full extent of each retina were analyzed by two experienced histopathologists. The retinal sections were coded by numbers so the pathologists were not aware of the treatment conditions. The severity of cytopathology was ranked on a scale of zero to +4 based on the degree of dendritic swelling in the inner plexiform layer and the degree of cytoplasmic swelling and nuclear changes in the neurons of the inner nuclear and ganglion cell layers, the specific neural elements of the retina that selectively undergo degeneration under either ischemic conditions or after exposure to a toxic concentration of Glu. The criteria for scoring were as follows:

Zero = total absence of cytopathology.

+ 1 = very slight cytopathology limited to mild swelling of inner plexiform dendritic processes.

+ 2 = more advanced neuronal degeneration with more conspicuous inner plexiform dendritic swelling plus mild to moderate cytoplasmic and nuclear changes in neurons of the inner nuclear and ganglion cell layers.

+ 3 = the same changes as in a 2 lesion but with 50 to 75% of the affected neurons showing more severe changes, signifying an advanced stage of neuronal degeneration.

+ 4 = the same changes as in a 3 lesion but with 75-100% of the affected neurons showing very severe changes, signifying uniformly severe cytopathological involvement across the entire retina.

The scores assigned by the two histopathologists for a given retina were usually in perfect agreement but varied in a few cases by a single rating unit. In such cases an average of the two readings was used to determine the final score.

The ratings for the various treatments are presented in Table 4. Differences between the control and experimental eyes were statistically analyzed by the Student's T test. The results indicate that either MK-801 or CNQX by itself provided an equivocal and highly variable amount of protection which could be described as partial at best. The two agents together provided a higher level of protection, with the majority of the retinas being in the 0 or +1 categories.

As mentioned above, this result was briefly summarized in Mosinger and Olney 1989b, which is an abstract rather than a full article. Since CNQX does not penetrate the BBB, this abstract did not offer any effective method of preventing severe ischemic damage inside those portions of the brain that are protected by the BBB.

## EXAMPLE 8: RAT RETINA TEST WITH NBQX AND MK-801

In a subsequent experunent, NBQX (which penetrates the BBB, as reported by Sheardown et al 1989) was tested in conjunction with MK-801 to assess their abilities, alone or in combination, to protect neurons in the adult rat retina against ischemic damage. These tests were performed using the same procedures and evaluation criteria described in Example 7, using photo-induced thrombosis to generate ischemic conditions in the retina. In each animal, one eye (which was injected with saline) served as the control, while the other eye (injected with NBQX and/or MK-801) was the experimental eye. All injections were of 7 microliters and were made into the vitreous humor of the eye.

NBQX was tested at only one dosage (60 nmole) because of solubility limitations as described above in conjunction with CNQX.

In these particular experiments, MK-801 was tested at 30 nmole per eye. In previous experiments, MK-801 had been tested by itself over a wide range of dosages (3, 10, 30, 60, and 160 nmole). The results of those prior tests indicated that the same level of protection was provided at all of those dosages. The data gathered from those tests indicates that MK-801 quickly reaches a "ceiling level" of protection, which is the maximal amount of protection it can provide by itself.

The data were gathered using the scale of damage that ranged from 0 for no detectable damage to +4 for extreme damage, as described above. Mean values were calculated for all similarly treated eyes, and the mean values for treated and untreated (saline control) eyes were compared.

The tests indicated that NBQX when administered by itself to 8 animals, reduced the level of damage

by about 30%, while MK-801, when administered by itself to 16 animals, reduced the level of damage by about 40%. By contrast, when NBQX and MK-801 were administered in conjunction with each other, they reduced the level of damage by more than 80%.

These results clearly indicate that an NMDA antagonist which penetrates the blood brain barrier, admimstered in conjunction with a non-NMDA antagonist which also penetrates the blood-brain barrier, can reduce neurotoxic ischemic damage in CNS neurons by a much greater level than either type of antagonist acting alone. These two classes of compounds, when used in combination, provide a higher degree of protection against ischemic damage than any other pharmacological agent or mixture previously reported, particularly in view of the comments made above about focal and global ischemia and the ability of the photothrombosis model to deprive the entire retina of its blood supply, thereby generating severe ischemic damage and offering a challenging model for evaluating protective agents.

When an anti-cholinergic agent as described above is also administered in conjunction with both the NMDA antagonist and the non-NMDA antagonist to prevent the neurotoxic side effects of the NMDA antagonist, the three-component mixture is believed to be the safest and most effective pharmacological agent ever discovered for reducing or preventing severe neurotoxic damage in the central nervous system due to hypoxia or ischemia.

Thus, there has been disclosed a class of pharmacological agents which can function safely and effectively in accomplishing beneficial results that were not previously available. Thiș in vention therefore satisfies all of the objectives set forth herein. Although this invention has been described with respect to specific embodiments, the details of these embodiments are not to be construed as limitations. Various equivalents and modifications may be made without departing from the spirit and scope of this invention, which is limited only by the claims that follow.

## REFERENCES


Adelman, G. (ed.), *Encyclopedia of Neurosciences* (Birkhauser, Boston, 1987)

Anis, N.A. et al, "The Dissociative Anaesthetics, Ketamine and Phencyclidine, Selectively Reduce Excitation of Central Mammalian Neurons by N-Methyl-Asparate", *Br. J. Pharmacol. 79* : 565(1983)

Berry, S.D., et al, "Stereoselective Effects of Two Phencyclidine Derivatives on N-Methylaspartate Excitation of Spinal Neurones in the Cat and Rat", *Eur. J Pharm. 96*: 261 (1983)

Boast, CA, "Neuroprotection after brain ischemia: role of competitive NMDA antagonists," *Neurology and Neurobiology 46*: 691-698(1988)

Braitman, D.J., et al, "MK-801 protects against seizures and brain damage induced by the cholinesterase inhibitor soman," *Neurosci. Abstr. 14*: 240(1988)

Burke, R.E., "The relative selectivity of anticholinergic drugs for the M1 and M2 muscarinic receptor subtypes," *Movement Disorders 1*: 135-144(1986)

Clifford, D.B., et al, "The functional anatomy and pathology of lithium-pilocarpine and high-dose pilocarpine seizures," *Neurosci. 23*: 953-968(1987)

Clifford, D.B., et al, "Ketamine and MK-801 prevent degeneration of thalamic neurons induced by focal cortical seizures," *Exp. Neurology 105*: 272-279(1989)

Das, M., et al, *Toxicol. Appl. Pharmacol. 39(1)*: 149-152(1977)

Drejer, J., et al., "A new potent glycine antagonist FG 9067 (MNQX) shows anti-convulsant activities," *J. Neurochem. 52*: Suppl., S42-D (1989).

Fleischhacker, W.W., et al, *J. Affective Disorder 12(2)*: 153-157(1987) Freedman, S.B., et al, "Muscarinic M1, M2 receptor binding; Relationship with functional efficacy," *Eur. J. Pharmacol. 156*: 133-142(1988)

Fuller, T.A. and Olney, J.W., "Only certain anti-convulsants protect against kainic acid neurotoxocity," *Neurobiol. Toxicol. and Teratol. 3*: 355-361(1981)

Goldman, M.E., et al, "Differentiation of [$^3$H]Phencyclidine and (+)-[$^3$H]SKF-10,047 Binding Sites in Rat Cerebral Cortex," *FEBS Lett. 170*: 333-336(1985)

Goodman, LS. and Gilman, A., *The Pharmacological Basis of Therapeutics* (5th ed., Macmillan, NY, 1975)

Gotti, B., et al., "Ifenprodil and SL 82.0715 as cerebral anti-ischemic agents. 1. Evidence for efficacy in models of focal cerebral ischemia," *J. Pharmacol and Exp. Therapeutics 247*: 1211-1221(1988)

Herrling, P.L, et al, "NMDA antagonistic properties of the enantiomers of CPP and CPP-ene," *Soc. Neurosci Abstr. 15*: 327(1989)

Hitri, A., et al, *Psychopharmacol. Bull. 23*(1): 33-37(1987)

Honchar, M.P., et al, "Systemic cholinergic agents induce seizures and brain damage in lithium-treated rats," *Science 220*: 323-325(1983)

Honore, T., et al, "Potent and competitive antagonism at non-NMDA receptors by FG 9041 and FG 9065," *Soc. Neurosci Abstr. 13*: 383(1987)

Honore, T., et al, "Quinoxalinediones: potent competitive non-NMDA glutamate receptor antagonists," *Science 241*: 701-703(1988)

Honore, T., et al, "Quisqualate receptor specific quinoxalinedione (FG 9202, NBQX) blocks kainate induced responses," *J. Neurochem. 52*: Suppl., S42-A (1989)

Kemp, J.D., et al, "Non-competitive antagonists of excitatory amino acid receptors," *Trends in Neurosci. 10*: 294(1987)

Kirino, T. and Sano, K, "Selective vulnerability in the gerbil hippocampus following ischemia," *Acta Neuropathol. 62*: 201-208(1984)

Labruyere, J., et al, "Phencyclidine and ketamine protect against seizure-related brain damage," *Neurosci. Abstr. 12*: 344(1986)

Labruyere, J., et al, "NMDA antagonists induce pathomorphological changes in cerebrocortical neurons," *Neurosci. Abst. 15*: 761(1989)

Langlais, P.J., et al, "Acute thiamine deficiency in the rat: Brain lesions, amino acid changes and MK-801 pretreatment," *Neurosci. Abst. 14*: 774(1988)

Lawrence, J.J., Fuller, T.A., and Olney, J.W., "MK-801 and PCP protect against ischemic neuronal degeneration in the gerbil hippocampus," *Neurosci. Abstr. 13*: 1079(1987)

Lodge, D., et al, "Excitatory amino acids and phencyclidine-like drugs," in Hicks, T.P., et al (Eds), *Excitatory Amino Acid Transmission* (Alan R. Liss, New York, 1987)

Mann, N., et al, *Arch Pharm. (Weinhein, Ger.) 309(4)*: 320-325(1976)

Maragos, W., et al, "High Correlation Between the Localization of [$^3$H]TCP Binding and NMDA Receptors," *Eur. J. Pharmacol. 123*: 173-174(1986)

McEvoy, J.P., et al, *Psychopharmacol. Bull. 23*(1): 30-32(1987)

McLeod, C.G., et al, "Acute neuropathology in soman-poisoned rats," *Neurotoxicology 5:* 53-58(1984)

Mosinger, I.L and Olney, J.W., "Photothrombosis-Induced Ischemic Neuronal Degeneration in the Rat Retina," *Exp. Neurol. 105*: 110-113(1989a)

Mosinger, J.L and Olney, J.W., "Combined Treatment with MK-801 and CNQX prevents Ischemic Neuronal Degeneration in the *In Vivo* Rat Retina," *Soc. Neurosci. Abstr. 15*: 45 (1989b)

Olney, J.W., "Glutamate-induced neuronal necrosis in the infant mouse hypothalamus: an electron microscopic study," *J. Neuropathol. Exp. Neurol. 30*: 75-90(1971)

Olney, J.W., "Excitotoxins: an overview," in *Excitotoxins*, Fuxe, K., et al, Eds. (Macmillan, London) pp 82-96(1983)

Olney, J.W., et al, "The Anti-Excitotoxic Effects of Certain Anesthetics, Analgesics and Sedative Hypnotics," *Neuroscience Letters 68*:29-34(1986)

Olney, J.W., et al, "Anti-Parkinsonian agents are phencyclidine agonists and N-methyl aspartate antagonists," *Eur. J. Pharmacol. 142*: 319-320(1987)

Olney, J.W., et al, "MK-801 powerfully protects against N-methyl aspartate neurotoxicity," *Eur. J. Pharmacol 141*: 357-361 (1987)

Olney, J. W., "Excitatory amino acids and neuropsychiatric disorders," *Biol. Psychiatry 26*: 505-525-(1989a)

Olney, J.W., et al, "MK-801 prevents hypobaric-ischemic neuronal degeneration in infant rat liver," *J. Neurosci. 9*:1701 (1989b)

Olney, J.W., et al, "Pathological changes induced in cerebrocortical neurons by phencyclidine and related drugs," *Science 244*: 1360-1362 (1989c)

*Physicians Desk Reference*, 40th Edition (Medical Economics Co., 1986)

Price, M.T., et al, "Procyclidine protects against soman neurotoxicity, even when administered after onset of convulsions," *Neurosci. Abst. 15*: 1349(1989).

Price, M.T., et al, "CNQX potently and selectively blocks kainate excitotoxicity in the chick embryo retina," *Soc. Neurosci. Abstr. 14*: 418(1988)

Pulsinelli, W.A. and Brierly, J.B., "A new model of bilateral hemispheric ischemia in the rat," *Stroke 10*: 267-272(1979)

Quilliam, M.A. and Wright, J.L.C., "The amnesic shellfish poisoning mystery," *Analytical Chemistry 61*: 1053-1059(1989)

Quirion, R., "Phencyclidine (Angel Dust)/Sigma 'Opiate' Receptor: Visualization by Tritium-Sensitive Film," *Proc. Nat'l. Acad. Sci.* USA. 78: 5881(1981)

Reif-Lehrer et al, "Effects of monosodium glutamate on chick embryo retina in culture," *Invest. Ophthalmol. 14*: 114-124(1975)

Rothman, S.M., and Olney, J.W., "Glutamate and the Pathophysiology of Hypoxia-Ischemic Brain Damage," *Annals of Neurology 19(2)*: 105-111 (1986)

Schroeder, C., et al, *Antiviral Res. Suppl. 1*: 95-99(1985)

Sheardown, M.J., et al, "NBQX, a specific non-NMDA receptor antagonist, shows neuroprotective effects against cerebral ischemia,"abstract published in Proceedings of the First International Conference on Therapy with Amino Acids and Analogs, Vienna, August 7-12, 1989. A full article is in Lubec and Rosenthal (eds.), Amino Acids: Chemistry, Biology, and Medicine (ESCOM Science Publishers, Leiden, Netherlands, January 1990).

Snell, L.D., et al, "Antagonism of NMDA-Induced Transmitter Release In The Rat Striatum By Phencyclidine-Like Drugs And Its Relationship To Turning Behaviour,"J. Pharmacol. Exp. Ther. 235: 50-56 (1985)

Syvalahti, E.X.G., et al, Pharmacol Toxicol. (Copenhagen) 60(1): 66-69 (1987)

Tamura, A., et al, "Focal cerebral ischemia in the rat ... following middle cerebral artery occlusion,"J. Cereb. Blood Flow Metab. 1: 53-60 (1981)

Turski, W.A., et al, "Limbic seizures produced by pilocarpine in rats,"Behav. Brain Research 9: 315-335 (1983)

Wong, E.H.F., et al, "The Anticonvulsant MK-801 Is A Potent N-Methyl-D-Aspartate Antagonist,"Proc. Nat'l. Acad. Sci U.S.A. 83: 7104-7108 (Sept. 1986)

MK-801 mentioned herein is (+)-5-methyl-10,11-dihydro-5H-dibenzo[a,d]cyclohepten-5,10-imine maleate. CGS 19755 is cis-4-phosphonomethyl-2-piperidinecarboxylic acid. CGP 37849 is 2-amino-4-methyl-5-phosphono-3-pentenoic acid. SL.820715 has the formula:

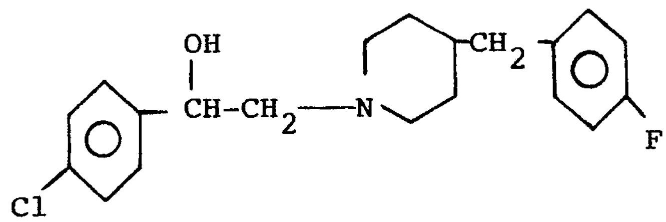

Table 1.

| PREVENTION OF MK-801-INDUCED VACUOLES BY ANTICHOLINERGIC AGENTS | | | | | | | | |
|---|---|---|---|---|---|---|---|---|
| Dose(mg/kg) of Anticholinergic | Percent of animals with MK-801-induced vacuoles | | | | | | | |
| | Scopolamine | Benztropine | Trihexyphenidyl | Atropine | Biperiden | Procyclidine | Benactyzine | Diphenhydramine |
| 25 | 0 | 0 | 0 | 0 | 0 | 0 | 0 | 0 |
| 10 | 0 | 0 | 0 | 0 | 0 | 0 | 0 | 100 |
| 5 | 0 | 0 | 0 | 0 | 25 | 50 | 100 | 100 |
| 2 | 0 | 0 | 50 | 50 | 50 | 50 | 100 | |
| 1 | 0 | 75 | 75 | 100 | 75 | 100 | | |
| 0.5 | 0 | 100 | 100 | 100 | 100 | | | |
| 2.25 | 0 | | | | | | | |
| 0.10 | 50 | | | | | | | |
| 0.05 | 100 | | | | | | | |
| 0(controls) | 100 | 100 | 100 | 100 | 100 | 100 | 100 | 100 |

All experimental animals received an anticholinergic drug 10 min after MK-801 (0.4 mg/kg sc). For each anticholinergic drug tested, a group of concurrent controls receiving only MK-801 (0.4 mg/kg sc) was included (n = 4 per treatment group).

TABLE 2

| RELATIVE AFFINITIES OF VARIOUS ANTI-CHOLINERGICS FOR M1 AND M2 RECEPTORS | | | |
|---|---|---|---|
| COMPOUND | $1/K_{app}$ M1 (brain) | $1/K_{app}$ M2 (heart) | M1/M2 RATIO |
| Scopolamine | 2.17 | 0.20 | 11.1 |
| Benztropine | 1.14 | 0.07 | 18.0 |
| Biperiden | 0.83 | 0.04 | 18.0 |
| Atropine | 0.63 | 0.29 | 2.1 |
| Trihexyphenidyl HCl | 0.45 | 0.03 | 13.0 |
| Dicyclomine | 0.20 | 0.006 | 33 |
| Pirenzepine | 0.07 | 0.001 | 48 |
| Source: | | | |
| Freedman et al, Eur. J. Pharmacology 156 : 133 at 136 (1988) | | | |

TABLE 3

| Potencies of Antagonists in Blocking NMDA, KA, Quis or Glu toxicity in Chick Embryo Retina | | | | |
|---|---|---|---|---|
| Compounds were rated according to the minimal concentration (μM) required to provide total protection against the neurotoxic effects of NMDA (120 μM) KA (25μM), Quis, (50 μM) or Glu (1000 μM). Antagonists were tested over a range of con entrations from 100 μM downward until a minimal effective concentration was established. | | | | |
| Potential antagonist | vs NMDA | vs KA | vs Quis | vs Glu |
| Competitive NMDA Antagonists | | | | |
| CPP | 15 | >100 | >100 | >100 |
| D-2-amino-5-phosphonopentanoate | 25 | >100 | >100 | >100 |
| D-2-amino-7-phosphonoheptanoate | 75 | >100 | >100 | >100 |
| Non-Competitive NMDA Antagonists | | | | |
| MK-801 | 0.2 | >100 | >100 | >100 |
| Phencyclidine | 0.5 | >100 | >100 | >100 |
| Ketamine | 5 | >100 | >100 | >100 |
| Tiletamine | 10 | >100 | >100 | >100 |
| Ifenprodil | 10 | >100 | >100 | >100 |
| Dextromethorphan | 50 | >100 | >100 | >100 |
| Non-NMDA Antagonists | | | | |
| CNQX | >100 | 50 | 15 | >100 |
| DNQX | >100 | 50 | 25 | >100 |

TABLE 4

| EFFICACIES OF CNQX AND MK-801 IN BLOCKING ISCHEMIC DAMAGE IN *IN VIVO* ADULT RAT RETINA | | | | |
|---|---|---|---|---|
| TREATMENT CNQX (nmol) | CONDITIONS MK-801 (nmol) | AVERAGE SCORES | | n |
| | | Control (± SEM) | Experimental (± SEM) | |
| 60 | - | 2.5 (± 0.29) | 1.72 (± 0.43) | 9 |
| - | 160 | 3.1 (± 0.23) | 1.67 (± 0.36) | 9 |
| - | 30 | 3.0 (± 0.28) | 1.59 (± 0.38) | 8 |
| 60 | 160 | 3.4 (± 0.18) | 0.63 (± 0.19) | 12 |
| 60 | 30 | 3.0 (± 0.21) | 0.50 (± 0.17) | 11 |

## Claims

1. Use of an anti-cholinergic agent which is capable of penetrating the blood-brain barrier and which exerts a pharmaceutically antagonistic effect on cholinergic receptors of the muscarinic type on the surfaces of neurons in the central nervous system in the preparation of a medicament for use in reducing the neurotoxic effects of a NMDA antagonist.

2. The use according to Claim 1 wherein the anti-cholinergic agent preferentially exerts a pharmaceutically antagonistic effect on cholinergic receptors of the M1 muscarinic type, compared to its effects on cholinergic receptors of the M2 muscarinic type, on the surfaces of neurons in the central nervous system.

3. The use according to Claim 1 wherein the anti-cholinergic agent is selected from scopolamine, atropine, benztropine, benactyzine, biperiden, triperiden, procyclidine, trihexyphenidyl, and diphenhydramine, and pharmaceutically acceptable salts and analogs thereof which function as anti-cholinergic agents.

4. A pharmaceutical agent suitable for use in reducing deleterious neurological effects in mammals, comprising a mixture of a NMDA antagonist and an anti-cholinergic agent, wherein the NMDA antagonist is present in a first concentration that can reduce excitotoxic damage in the brain, and wherein the anti-cholinergic agent is present in a second concentration that can reduce one or more neurotoxic effects which otherwise would be caused by the NMDA antagonist if administered to the mammal without an accompanying anti-cholinergic agent.

5. A pharmaceutical agent according to Claim 4 wherein the anti-cholinergic agent exerts a pharmaceutically antagonistic effect on cholinergic receptors of the M1 muscarinic type on the surfaces of neurons in the central nervous system.

6. A pharmaceutical agent according to Claim 4 wherein the anti-cholinergic agent is selected from scopolamine, atropine, benztropine, benactyzine, biperiden, triperiden, procyclidine, trihexyphenidyl, and diphenhydramine, and pharmaceutically acceptable salts and analogs thereof which function as anti-cholinergic agents.

7. An article of manufacture comprising packaging material and an anti-cholinergic agent contained within the packaging material, wherein the anti-cholinergic agent is therapeutically effective for reducing one or more neurotoxic effects of NMDA antagonists, and wherein the packaging material comprises a label which indicates that the anti-cholinergic agent can be used for reducing one or more neurotoxic effects of NMDA antagonists.

8. An article according to Claim 7 wherein the anti-cholinergic agent is selected from scopolamine, atropine, benztropine, benactyzine, biperiden, triperiden, procyclidine, trihexyphenidyl, and diphenhydramine, and pharmaceutically acceptable salts and analogs thereof which function as anti-cholinergic agents.

9. An article according to Claim 7 or 8 wherein the packaging material also contains a NHDA antagonist.

10. Products containing a NHDA antagonist and an anti-cholinergic agent which is capable of penetrating the blood-brain barrier and which exerts a pharmaceutically antagonistic effect on cholinergic receptors of the muscarinic type on the surfaces of neurons in the central nervous system as a combined preparation for simultaneous, separate or sequential use in neurological therapy to protect neurons inside the central nervous system.

11. Use of a NMDA antagonist which penetrates blood-brain barriers in quantities sufficient to inhibit the functioning of NMDA receptors in the preparation of a medicament for use in combination with a non-NMDA antagonist which penetrates blood-brain barriers in quantities sufficient to inhibit the functioning of at least one type of non-NMDA receptor in reducing excitotoxic damage to neurons in a mammalian central nervous system.

12. Use of a non-NMDA antagonist which penetrates blood-brain barriers in quantities sufficient to inhibit the functioning of at least one type of non-NMDA receptor in the preparation of a medicament for use in combination with a NMDA antagonist which penetrates blood-brain barriers in quantities sufficient to inhibit the functioning of NMDA receptors in reducing excitotoxic damage to neurons in a mammalian central nervous system.

13. The use according to Claim 11 or 12 wherein the non-NMDA antagonist comprises a quinoxalinedione derivative.

14. A pharmaceutical agent comprising a mixture of a NMDA antagonist and a non-NMDA antagonist, wherein both antagonists penetrate blood-brain barriers in quantities sufficient to inhibit the functioning of excitatory amino acid receptors, and wherein the mixture can reduce excitotoxic damage to neurons in a mammalian central nervous system more effectively than a NMDA antagonist alone.

15. Use of an anti-cholinergic agent which can penetrate the blood-brain barrier and which is effective for reducing one or more neurotoxic effects on NMDA antagonists in the preparation of a medicament for use in combination with a NMDA antagonist which penetrates blood-brain barriers in quantities sufficient to inhibit the functioning of NMDA receptors and a non-NHDA antagonist which penetrates blood-brain barriers in quantities sufficient to inhibit the functioning of at least one type of non-NMDA receptor in reducing excitotoxic to neurons in a mammalian central nervous system.

16. A pharmaceutical agent comprising:

(a) a NMDA antagonist which penetrates blood-brain barriers in quantities sufficient to inhibit the functioning of NMDA receptors;

(b) a non-NMDA antagonist which penetrates blood-brain barriers in quantities sufficient in inhibit the functioning of at least one type of non-NMDA receptor; and

(c) an anti-cholinergic agent which penetrates the blood-brain barrier and which is effective for reducing one or more neurotoxic effects of NMDA antagonists.

17. An article of manufacture, comprising packaging material and a pharmaceutical agent contained within the packaging material, wherein the pharmaceutical agent comprises:

(a) a NMDA antagonist which penetrates blood-brain barriers in quantities sufficient to inhibit the functioning of NMDA receptors;

(b) a non-NMDA antagonist which penetrates blood-brain barriers in quantities sufficient to inhibit the functioning of at least one type of non-NMDA receptor; and

(c) an anti-cholinergic agent which penetrates the blood-brain barrier,

wherein the anti-cholinergic agent is effective for reducing neurotoxic side-effects of the NMDA antagonist, and wherein the packaging material comprises a label which indicates that the pharmaceutical agent can be used for reducing excitotoxic damage to neurons.

18. Products containing a NMDA antagonist which penetrates blood-brain barriers in quantities sufficient to inhibit the functioning of NMDA receptors and a non-NMDA antagonist which penetrates blood-brain barriers in quantities sufficient to inhibit the functioning of at least one type of non-NMDA receptor as a combined preparation for simultaneous, separate or sequential use in reducing excitotoxic damage to neurons in a mammalian central nervous system.

19. Products according to Claim 15, further containing an anti-cholinergic agent which can penetrate the blood-brain barrier and which is effective for reducing one or more neurotoxic effects of NMDA antagonists.